# EUROPÄISCHE PATENTSCHRIFT

(11) **EP 1 916 912 B1**
(45) Veröffentlichungstag und Bekanntmachung des Hinweises auf die Patenterteilung: **13.06.2012**
(21) Anmeldenummer: 06776812.7
(22) Anmeldetag: 12.08.2006
(51) Int. Cl.: A23L 1/30

(54) **PULVERFÖRMIGE STEROLFORMULIERUNGEN MIT KOLLOIDBILDNERN**
POWDERY STEROL FORMULATIONS COMPRISING COLLOID-FORMING AGENTS
FORMULATIONS DE STEROLS PULVERULENTES CONTENANT DES FORMATEURS DE COLLOIDES

(30) Priorität: 23.08.2005 DE 102005039835
(43) Veröffentlichungstag der Anmeldung: 07.05.2008
(73) Patentinhaber: Cognis IP Management GmbH, 40589 Düsseldorf (DE)
(72) Erfinder: HORLACHER, Peter, 89287 Bellenberg (DE); SALACZ, Robert, 89250 Senden (DE); SCHWARZER, Jörg, 40723 Hilden (DE); GIERKE, Jürgen, 89257 Illertissen/Betlinshausen (DE)
(86) Internationale Anmeldenummer: PCT/EP2006/007998
(87) Internationale Veröffentlichungsnummer: WO 2007/022891

(56) Entgegenhaltungen:
- EP-A1- 0 947 197
- WO-A2-02/43506
- WO-A2-03/105611
- JP-A- 56 142 212
- JP-A- 2000 119 686
- US-A- 6 054 144
- US-A1- 2004 033 202
- US-A1- 2006 034 934
- DATABASE WPI Week 199134 Derwent Publications Ltd., London, GB; AN 1991-248698 XP002404845 & JP 03 161448 A (ASAHI CHEM IND CO LTD) 11. Juli 1991 (1991-07-11)

## Beschreibung

### Gebiet der Erfindung

Die Erfindung befindet sich auf dem Gebiet der Lebensmittel und betrifft Formulierungen zur Einarbeitung in Lebensmittel, kosmetische und pharmazeutische Zubereitungen, die Sterole, Stanole und/oder deren Ester enthalten, ein Verfahren zur Herstellung derselben sowie Zubereitungen, insbesondere Lebensmittel, die diese Formulierungen beinhalten.

### Stand der Technik

Der Zusatz von Sterolen und Stanolen in Lebensmitteln aufgrund ihrer cholesterinsenkenden Eigenschaften und dadurch bedingten Prävention von Folgekrankheiten wie Atherosklerose, Herzerkrankungen oder Bluthochdruck, ist seit Jahren bekannt.

Da Phytosterole und -stanole in Wasser unlöslich und in Fetten und Ölen nur schlecht löslich sind, wirft die Einarbeitung der Cholesterinsenker in Lebensmittelzubereitungen, kosmetische oder pharmazeutische Produkte erhebliche Probleme auf. Aus dem ungünstigen Löslichkeitsverhalten der Substanzen resultiert neben der schlechten Dispergierbarkeit auch eine verminderte Bioverfügbarkeit und eine unbefriedigende Stabilität der Lebensmittelzubereitungen. Der Stand der Technik bietet daher zahlreiche Formulierungsvorschläge zur Lösung dieser Probleme an.

Zu den Bemühungen, die Löslichkeit zu erhöhen, gehörte die Formulierung von Estern der Sterole beispielsweise beansprucht in der Europäischen Patentanmeldung EP 1275309 A1 oder Estern der Stanole wie im US-Patent US 5,502,045 beschrieben, die durch ihre bessere Löslichkeit eine geringfügig verbesserte Verarbeitbarkeit aufwiesen jedoch gegenüber den freien Sterolen auch eine veränderte hypocholesterinämische Wirkung aufwiesen.

In zahlreichen Patentanmeldungen wird beschrieben, wie die Verfügbarkeit von Sterolen über die Reduktion der Partikelgrößen vornehmlich durch Mikronisation verbessert werden kann. Einen Prozess zur Herstellung einer Steroldispersion, in der die Partikelgrößenverteilung der Sterole bei 0,1 bis 30 µm liegt entnimmt man der Internationalen Anmeldung WO 03/105611 A2. Zubereitete Dispersionen sind jedoch aufgrund der Sedimentation oder des Verkeimungsrisikos nicht lange lagerstabil.

Häufig ist die Mikronisation der Sterolpartikel alleine nicht ausreichend, um eine gute Einarbeitbarkeit zu erreichen. Zwar lässt sich durch die Erhöhung der Oberfläche die Bioverfügbarkeit der fein dispergierten Teilchen verbessern, jedoch sind gerade die mikronisierten Partikel schlecht benetzbar, aggregieren leicht und schwimmen auf wässrigen Oberflächen meistens auf. Oft lässt sich das gemahlene Sterol nur mit speziellen Methoden in einem Getränk dispergieren, wozu intensives Mischen notwendig ist. Diese Geräte stehen jedoch im Normalfall dem Endanwender der Lebensmittelhersteller nicht zur Verfügung.

Daher kombinieren viele Hersteller die Mikronisation der Sterole mit der zusätzlichen Anwendung von Emulgatoren. Ein Beispiel dafür sind die in dem Europäischen Patent EP 0897671 B1 beanspruchten Zubereitungen mit Sterolen und Sterolestern mit einer Partikelgröße von maximal 15 µm in einem Gemisch mit Emulgatoren, wobei das Gewichtsverhältnis Emulgator zu Sterol in der wässrigen Phase weniger als 1:2 ist. Häufig verwendete Emulgatoren sind Monoglyceride und Polysorbate [US 6,623,780 B1, US 6,376,482 B2, WO 02/28204 A1]. In der Internationalen Patentanmeldung WO 03/086468 A1 werden pulverförmige Sterolesterformulierungen mit einem geringen Proteingehalt und ebenfalls Mono- und Diglyceriden als Emulgatoren offenbart. Auch wenn diese sich durch eine gute Verträglichkeit auszeichnen und als Lebensmittelemulgatoren über einen langen Zeitraum bereits bekannt sind, versucht man die Menge der Emulgatoren zu verringern oder sie gar ganz zu vermeiden, da Emulgatoren auch die Bioverfügbarkeit weiterer in den Lebensmitteln vorhandener Substanzen beeinflussen oder die Stabilität der Formulierungen negativ verändern können.

Die Vermeidung von Emulgatoren war auch Ziel der in dem Europäischen Patent EP 1059851 B1 offenbarten wasserhaltige Sterolformulierungen, die Verdicker zur besseren Dispergierbarkeit enthalten. In der Internationalen Patentanmeldung WO 98/13023 werden mikronisierte Sterole auf Basis von Zuckern in wäßrigen Dispersionen verarbeitet. Formulierungen, die wässrige Suspensionen darstellen, weisen jedoch in der Regel eine geringe Lagerstabilität hinsichtlich ihrer physiko-chemischen und mikrobiologischen Eigenschaften auf. Diese Probleme bei der Lagerung lassen sich durch die Verwendung von trockenen Formulierungen vermeiden.

Zahlreiche Methoden zur Verbesserung der Löslichkeit und Dispergierbarkeit - so auch die Formulierung trockener Pulver - werden in der Internationalen Patentanmeldung WO 99/63841 A1 vorgestellt. Als Träger werden PEG, PVP, Copolymere, Celluloseether und -ester vorgeschlagen. Auch der direkte Einsatz von Lebensmittelgrundstoffen als Träger für pulverisierte Sterole ist aus der EP 1 003 388 B1 bekannt.

Bei dem Einsatz trockener Pulver, die lipophile Inhaltsstoffe enthalten, steht jedoch die Problematik der Dispergierbarkeit im Vordergrund.

Gerade bei diesen Formulierungen ist daher meistens die Einarbeitung weiterer Hilfsstoffe, in der Regel Emulgatoren notwendig. Gut dispergierbare Sterolhaltige Pulverformulierungen wurden in der Europäischen Patentschrift EP 0 947 197 B1**,** der Internationalen Patentanmeldung WO 03/000075 A1 und im US Patent US 5,932,562 beschrieben. Als Träger diente hier Maltodextrin, das jedoch nicht ohne den Einsatz von Emulgatoren verwendet werden konnte.

Auch die deutsche Offenlegungsschrift DE 102 53 111 A1 offenbart pulverförmige Phytosterol-Formulierungen mit einer mittleren Teilchengröße von 0,01 bis 100 µm, die sich auch ohne Anwendung von Emulgatoren gut in Wasser redispergieren lassen. Mit Hilfe von Schutzkolloiden, insbesondere modifizierter Stärke oder Natrium-Caseinat, die allerdings mit Weichmachern wie Saccharose kombiniert werden, werden gut dispergierbare Pulver durch Sprühtrocknung hergestellt. Ein Nachteil der zusätzlichen Anwendung von Zuckern als Weichmacher ergibt sich aus der fehlenden Geschmacksneutralität der Sterolformulierungen, die die spätere Anwendung in unterschiedlichen Lebensmitteln wiederum einschränkt.

Aufgabe der vorliegenden Erfindung war es, eine Formulierung zur Verfugung zu stellen, die eine einfache und gute Dispergierung und Einarbeitung von Sterolen und Stanolen in Lebensmitteln ermöglicht, wobei die Anwendung von Emulgatoren reduziert oder ganz vermieden werden soll. Diese Sterolformulierung sollte einfach herzustellen sein und sich durch eine gute Lagerstabilität und Geschmacksneutralität auszeichnen.

### Beschreibung der Erfindung

Gegenstand der Erfindung sind pulverförmige Zubereitung nach Anspruch 1.

Die erfindungsgemäßen Formulierungen zeichnen sich durch gute Solubilisierungseigenschaften, verminderte Aggregations- und Agglomerationseigenschaften und eine verbesserte Benetzbarkeit aus und ermöglichen so eine einfache Dispergierung von Sterolen, Stanolen und deren Estern in wasser- und fetthaltigen Zubereitungen. Sie lassen sich hervorragend handhaben, da sie ohne aufwendige Ausrüstung weiterverarbeitet werden können, zu geschmacksneutralen sterolhaltigen Mitteln führen und eine gute Lagerstabilität zeigen.

Die erfindungsgemäßen Zubereitungen können auf einfache Weise in Lebensmittel, insbesondere in Milch, Milchgetränke, Molke-, Joghurtgetränke, Margarine, Fruchtsäfte, Fruchtsaftgemische, Fruchtsaftgetränke, Gemüsesäfte, Kohlensäurehaltige und Kohlensäurefreie Getränke, Sojamilchgetränke oder proteinreiche flüssige Nahrungsersatzgetränke, sowie fermentierte Milchzubereitungen, Joghurt, Trinkjoghurt, oder Käsezubereitungen, aber auch in kosmetische oder pharmazeutische Zubereitungen eingearbeitet werden.

Vorzugsweise können die erfindungsgemäßen Zubereitungen sogar ohne Emulgatoren hergestellt werden. Durch den Verzicht auf den Einsatz von Emulgatoren kann das Auftreten von Unverträglichkeitsreaktionen mit weiteren Hilfsstoffen verringert und die Lagerstabilität der Preformulierung entscheidend verbessert werden.

Der Ausdruck "emulgatorfrei" bedeutet, dass insbesondere der Einsatz von üblichen Lebensmittelemulgatoren wie Lecithinen, Monoglyceriden, Diglyceriden, Polysorbaten, Natriumstearyllactylat, Glycerolmonostearat, Milchsäureestern und Polyglycerinestern vermieden wird. Natürlich in den Lebensmitteln vorkommende Emulgatoren wie beispielsweise Cholesterol können selbstverständlich in der Preformulierung nicht verhindert werden, jedoch soll während der Herstellung der erfindungsgemäßen Sterol-/Stanolzubereitungen kein Emulgator als Hilfsstoff zugefügt werden.

### Sterole und/oder Stanole

In der vorliegenden Erfindung werden die aus Pflanzen und pflanzlichen Rohstoffen gewonnenen Sterole, sogenannte Phytosterole und Phytostanole eingesetzt. Bekannte Beispiele sind Ergosterol, Brassicasterol, Campesterol, Avenasterol, Desmosterol, Clionasterol, Stigmasterol, Poriferasterol, Chalinosterol, Sitosterol und deren Mischungen, darunter werden bevorzugt β- Sitosterol und Campesterol verwendet. Ebenso fallen die hydrierten gesättigten Formen der Sterole, der sogenannten Stanole unter die eingesetzten Verbindungen, auch hier werden β-Sitostanol und Campestanol bevorzugt.

Als pflanzliche Rohstoffquellen dienen unter anderem Samen und Öle von Sojabohnen, Kanola, Palmkernen, Mais, Kokos, Raps, Zuckerrohr, Sonnenblume, Olive, Baumwolle, Soja, Erdnuss oder Produkte aus der Tallölproduktion.

Es werden außerdem Veresterungsprodukte der Sterole und Stanole, vorzugsweise mit gesättigten und/oder ungesättigten Fettsäuren mit 6 bis 22 und vorzugsweise 12 bis 18 Kohlenstoffatomen verarbeitet. Die Erfindung ist jedoch nicht begrenzt auf diese Art der Ester. So sind auch Phenolsäureester, insbesondere Derivate der Zimtsäure, Kaffeesäure und Ferulasäure einsetzbar.

Dabei können natürlich vorkommende Ester aus pflanzlichen Rohstoffen direkt gewonnen werden, oder die Sterol-/Stanolester werden durch Umesterung mit anderen Estern hergestellt. Ebenso können Derivate eingesetzt werden, die durch Veresterung freier Sterole oder Stanole mit den entsprechenden Fettsäuren entstanden sind.

Die erfindungsgemäßen Zubereitungen enthalten jedoch bevorzugt die freien, unveresterten Sterole und Stanole, da die veresterten Derivate bereits eine verbesserte Löslichkeit und Dispergierbarkeit gegenüber den freien Sterolen und Stanolen aufweisen.

Die pulverförmigen Zubereitungen enthalten 30 bis 50 Gew. %, bevorzugt 35 bis 45 Gew. % Sterole, Stanole und/oder Sterol- und Stanolester bezogen auf die emulgatorfreie pulverförmige Zubereitung.

Ein weiterer Gegenstand der Erfindung sind daher Zubereitungen, die die Sterol-/Stanol(ester)formulierungen der genannten Zusammensetzung enthalten. Sie werden bevorzugt in Getränken und Milchprodukten eingesetzt, die dann 0,1 bis 50 Gew. %, bevorzugt 1 bis 20 Gew. % der pulverförmigen Zubereitungen bezogen auf das Gesamtgewicht der Lebensmittel, enthalten.

### Natürliche makromolekulare Kolloidbildner

Die für die Erfindung natürlichen makromolekularen Kolloidbildner ist Gummi arabicum.

Der ausgewählte Kolloidbildner zeichnet sich durch eine niedrige Viskosität nach der Quellung in Wasser aus. Da vorzugsweise in den Formulierungen schlecht lösliche Sterole und/oder Stanole eingesetzt werden, ist man bei der Verwendung der Träger auf niederviskose Materialien beschränkt. In Versuchen hatte sich gezeigt, dass hochviskose Materialien wie beispielsweise modifizierte Stärken und Stärkederivate keine stabile Dispersion liefern.

Das erfindungsgemäße Mengenverhältnis a) Sterole und/oder Stanole und/oder deren Ester zu b) natürliche makromolekulare Kolloidbildner von 30:70 bis 50:50 ergab sich x ebenfalls durch die Auswahl der Trägermaterialien. Werden zu hohe Mengen an Kolloidbildnem eingesetzt, nimmt die Stabilität der Dispersion ab, die Sprüheigenschaften der Dispersion bei der Herstellung des Pulvers verschlechtern sich und die guten Dispergiereigenschaften der Endformulierung in den unterschiedlichen Lebensmitteln nehmen ab. Werden zu geringe Mengen an Trägermaterialien eingesetzt, nimmt die Benetzungsfähigkeit des Pulvers und somit auch die gute Einarbeitbarkeit in Lebensmitteln ab. Bevorzugt wird daher ein Verhältnis von a) zu b) von 35:65 bis 50-50 und besonders bevorzugt von 35 : 65 bis 50:50

Bei den besonders bevorzugten Formulierungen fällt der Gehalt an weiteren Hilfsstoffen in der sterolhaltigen Pulverformulierung gering aus, daraus ergibt sich, dass die erfindungsgemäßen Zubereitungen vorzugsweise 50 bis 70 Gew. %, besonders bevorzugt 55 bis 65 Gew. % natürliche makromolekulare Kolloidbildner bezogen auf die pulverförmige Zubereitung enthalten, als am besten geeignet hat sich Gummi arabicum erwiesen.

### Gummi arabicum

Gummi arabicum ist ein natürlich vorkommendes Polysaccharid mit einem mittleren Molekulargewicht von 260 000 bis 1160000 Dalton, das eine Mischung aus den Calcium-, Magnesium- und Kaliumsalzen von Polyarabinsäure darstellt. Zu den Bausteinen der Arabinsäure zählen D-Galaktose, L-Rhamnose, L-Arabinose und D-Glucuronsäure. (1,3)-verknüpfte β-D-Galactopyranosylreste bilden die Hauptkette, die durch (1,6)- Verbindung verzweigte Seitenketten enthält.

Es wird aus dem Pflanzensaft verschiedener Akazien (Acacia senegal, Acacia seyal) und Mimosenarten dadurch gewonnen, dass man die Rinde der Pflanzen einschneidet und die an der Luft trocknenden Tropfen des Pflanzensaftes, die zum Verschluss der Wunde vom Baum ausgeschieden werden, aberntet.

Der Einsatz von Gummi arabicum ist in der Lebensmittelindustrie und besonders in der Getränkeindustrie zur Stabilisierung von Geschmacksstoffen und essenziellen Ölen weit verbreitet. Es gehört ernährungsphysiologisch zu den Ballaststoffen und hat einen niedrigen Energiewert - beispielsweise die Hälfte des Energiewertes von Stärke oder Maltodextrin. Daher eignet sich Gummi arabicum besonders zur Herstellung der erfindungsgemäßen sterolhaltigen Pulver, die gerade in Getränken eingesetzt werden sollen.

### Herstellung

Die erfindungsgemäßen pulverförmigen Sterol-/Stanol(ester)formulierungen sind erhältlich dadurch , dass man
a) gemahlene Sterole und/oder Stanole und/oder deren Ester in Wasser von 80 bis 100°C durch intensives Rühren dispergiert
b) der Dispersion natürliche makromolekulare Kolloidbildner ausgewählt aus der Gruppe, die gebildet wird von Gummi arabicum, Gelatine, Traganth, Alginaten, Carragen, Guargummi und Xanthan in einem Mengenverhältnis a) zu b) von 30:70 bis 50:50 .Gummi arabicum als trockenes Pulver zugibt, 30:70
c) die Dispersion nachfolgend unter hohem Druck homogenisiert
d) und die so homogenisierte Dispersion sprühtrocknet.

Vorzugsweise werden im Schritt a) mikronisierte Sterole und Stanole eingesetzt mit einer mittleren Teilchengröße von 0,01 bis 50 µm, bevorzugt 0,05 bis 30 µm und besonders bevorzugt 0,1 bis 10 µm. Als natürlicher makromolekularer Kolloidbildner wird bevorzugt Gummi arabicum eingesetzt.

Der Schritt d) ermöglicht es, dass man auf die Verwendung von Emulgatoren verzichten kann, da die Sprühtrocknung zu einer Umhüllung der in wässrigen Systemen schlecht dispergierbaren Sterole und/oder Stanole mit den Trägermaterialien führt und durch die dann hydrophile Oberfläche eine Einarbeitung insbesondere in wässrige Systeme entscheidend verbessert wird.

Ein weiterer Gegenstand der Erfindung ist daher auch ein Verfahren zur Herstellung von sterolhaltigen Pulvern, bei dem man
a) mikronisierte Sterole und/oder Stanole in Wasser von 80 bis 100°C durch intensives Rühren dispergiert,
b) der Dispersion natürliche makromolekulare Kolloidbildner ausgewählt aus der Gruppe, die gebildet wird von Gummi arabicum, Gelatine, Traganth, Alginaten, Carragen, Guargummi und Xanthan in einem Mengenverhältnis a) zu b) von 30:70 bis 50:50 Gummi arabicum als trockenes Pulver zugibt,
c) diese Dispersion nachfolgend unter hohem Druck homogenisiert und
d) die homogenisierte Dispersion sprühtrocknet.

### Teilchengröße

Die Teilchengrößenverteilung der erfindungsgemäßen Zubereitung ist abhängig vom Herstellungsverfahren. Bei der bevorzugten Sprühtrocknung liegt die mittlere Teilchengröße in der Regel zwischen 0,01 bis 50 µm, vorzugsweise zwischen 0,1 bis 30 µm und besonders bevorzugt m Bereich von 1 bis 10 µm.

Die Teilchengrößenverteilung wurde mit einem Gerät der Fa. Beckman Coulter, Typ LS 230 unter Nutzung des optischen Models Emulsion.rfd PIDS inclded (vom 14.08.01) nach Bedienungsanleitung (1994) bestimmt. Als Messmedium wurde Wasser verwendet. Die Teilchengrößenmessungen erfolgten unmittelbar nach der Herstellung der Dispersionen. Ausgewählte Dispersionen wurden einem Lagertest (siehe Beispiele) unterzogen.

### Beispiele

1650 g Wasser wurden auf ca. 90°C aufgeheizt. Hierzu wurden 200 g Generol 122 NG (gemahlenes Sterin Fa. Cognis Deutschland GmbH & Co. KG) unter intensivem Rühren gegeben und so lange gerührt bis eine feine Verteilung vorlag. Anschließend wurden 319 g Gummi Arabicum (TS 94%) zugegeben. Der Ansatz wurde auf 75°C abgekühlt und die Dispersion homogenisiert (bei 220/30 bar im Homogenisator Typ LAB 100/60 der Firma Schröder) und sprühgetrocknet (APV Anhydro Typ 3 S).
Sprühbedingungen:

| | |
|---|---|
| Temperatureinlass: | 185°C |
| Temperaturauslass: | 93°C |
| Zerstäuber: | 24000 UpM |

Es resultierte ein gut in Wasser, Milch und Orangensaft bei Raumtemperatur einrührbares Pulver, das in einer Konzentration von 1 Gew. % bezogen auf das sterolhaltige Getränk keinen unangenehmen Geschmack während und nach der Aufnahme des Getränkes aufwies. Die Messung der Teilchengrößenverteilung des Pulvers - gemessen durch Laserdiffraktometrie (Fa. Beckman Coulter, Typ LS 230) ergab einen d50 von 5 µm und einen d90 von 29µm.

Der Versuch wurde in folgender Zusammensetzung wiederholt:

| | |
|---|---|
| Pulver 1: | 40% Sojasterin und 60% Gummi Arabicum |
| Pulver 2: | 40% Tall/Raps-Sterin (80/20) und 60% Gummi Arabicum |
| Pulver 3: | 40% Tall/Raps-Sterin und 30% Gummi Arabicum und 30% Maltodextrin |
| Pulver 4: | 40% Tall/Raps-Sterin und 60% Maltodextrin |

Pulver 3 und 4 sind Vergleichsbeispiele.

Eindispergiert in unten angegebenem System bei 600 rpm gerührt (0,4% und 1 % Steringehalt)

| | Pulver 1 | Pulver 2 | Pulver 3 | Pulver 4 |
|---|---|---|---|---|
| Wasser 15°C | gut einrührbar | gut einrührbar | einrührbar | bedingt einrührbar |
| Wasser 60°C | gut einrührbar | gut einrührbar | gut einrührbar | bedingt einrührbar |
| Milch 18°C | gut einrührbar | gut einrührbar | gut einrührbar | einrührbar |
| O-Saft 18°C | gut einrührbar | gut einrührbar | einrührbar | bedingt einrührbar |

Die sensorische Bewertung ergab, dass die Pulver 1 und 2 in Wasser neutral schmeckten und dass sowohl Milch als auch O-Saft als sehr gut bewertet wurden. Es konnte kein typischer Steringeschmack detektiert werden.

Es ergab sich ein deutlicher Unterschied zur Kolloidbildnerfreien Sterindispersion und den Pulvern 3 und 4, die sensorisch schlechter abschnitten.

## Patentansprüche

1. Pulverförmige Zubereitung, bestehend aus
a) 30 bis 50 Gew.% Sterolen und/oder Stanolen und/oder deren Estern und
b) 50 bis 70 Gew.% Gummi arabicum
bezogen auf das Gesamtgewicht der Zubereitung.

2. Pulverförmige Zubereitung gemäß Anspruch 1, **dadurch gekennzeichnet, dass** sie als Komponente a) unveresterte Sterole und/oder Stanole enthält.

3. Pulverförmige Zubereitung, gemäß den Ansprüchen 1 und/oder 2, **dadurch gekennzeichnet, dass** sie eine mittlere Teilchengröße von 0,01 bis 50 µm aufweist.

4. Lebensmittel, enthaltend 0,1 bis 50 Gew. % der pulverförmigen Zubereitung gemäß der Ansprüche 1 bis 3 bezogen auf das Gesamtgewicht der sterol/stanolhaltigen Lebensmittel.

5. Getränke und Milchprodukte, enthaltend 0,1 bis 50 Gew. % der pulverförmigen Zubereitung gemäß der Ansprüche 1 bis 4 bezogen auf das Gesamtgewicht der sterolstanolhaltigen Getränke und Milchprodukte.

6. Verfahren zur Herstellung von sterolhaltigen Pulvern nach Anspruch 1, bei dem man
a) mikronisierte Sterole und/oder Stanole in Wasser von 80 bis 100°C durch intensives Rühren dispergiert
b) der Dispersion Gummi arabicum in einem Mengenverhältnis a) zu b) von 30:70 bis 50:50 als trockenes Pulver zugibt
c) diese Dispersion nachfolgend unter hohem Druck homogenisiert
d) und die homogenisierte Dispersion sprühtrocknet.

## Claims

1. Powder-form preparation containing
a) 30 to 50% by weight sterols and/or stanols and/or esters thereof and
b) 50 to 70% by weight gum arabic,
based on the total weight of the preparation.

2. Powder-form preparation as claimed in claim 1, **characterized in that** it contains unesterified sterols and/or stanols as component a).

3. Powder-form preparation as claimed in claims 1 and/or 2, **characterized in that** it has a mean particle size of 0.01 to 50 µm.

4. Food containing 0.1 to 50% by weight of the powder-form preparation as claimed in claims 1 to 3, based on the total weight of the sterol/stanol-containing food.

5. Beverages and milk products containing 0.1 to 50% by weight of the powder-form preparation as claimed in claims 1 to 4, based on the total weight of the sterol/stanol-containing beverages and milk products.

6. Process for the production of sterol-containing powders as claimed in claim 1, which comprises the steps of
a) dispersing micronized sterols and/or stanols in water heated to 80 to 100°C by intensive stirring,
b) adding gum arabic as a dry powder, to the dispersion in a quantity ratio of a) to b) of 30:70 to 50:50,
c) then homogenizing this dispersion under high pressure and
d) spray drying the homogenized dispersion.

## Revendications

1. Préparation pulvérulente, constituée de
a) 30 à 50 % en poids de stérols et/ou de stanols et/ou de leurs esters et
b) 50 à 70 % en poids de gomme arabique
par rapport au poids total de la préparation.

2. Préparation pulvérulente selon la revendication 1, **caractérisée en ce qu'**elle contient en tant que composant a) des stérols et/ou stanols non estérifiés.

3. Préparation pulvérulente selon les revendications 1 et/ou 2, **caractérisée en ce qu'**elle présente une taille moyenne de particule de 0,01 à 50 µm.

4. Produits alimentaires, contenant de 0,1 à 50 % en poids de la préparation pulvérulente selon les revendications 1 à 3, par rapport au poids total des produits alimentaires contenant des stérols/stanols.

5. Boissons et produits laitiers, contenant de 0,1 à 50 % en poids de la préparation pulvérulente selon les revendications 1 à 4, par rapport au poids total des boissons et produits laitiers contenant des stérols/stanols.

6. Procédé pour la fabrication de poudres contenant des stérols selon la revendication 1, dans lequel
a) on disperse par vigoureuse agitation des stérols et/ou stanols dans de l'eau à une température de 80 à 100 °C
b) on ajoute à la dispersion de la gomme arabique en un rapport quantitatif de a) à b) de 30:70 à 50:50 sous forme de poudre sèche
c) ensuite on homogénéise sous haute pression cette dispersion
d) et on sèche par atomisation la dispersion homogénéisée.
